# EUROPÄISCHE PATENTANMELDUNG

(11) **EP 2 452 623 A1**
(43) Veröffentlichungstag der Anmeldung: **16.05.2012**
(21) Anmeldenummer: 11155043.0
(22) Anmeldetag: 18.02.2011
(51) Int. Cl.: A61B 5/107, A61B 19/00, A61B 5/11

(54) **Abbildungsvorrichtung zur großflächigen Abbildung eines Körperabschnitts**

(30) Priorität: 15.11.2010 DE 102010051475; 16.11.2010 DE 102010051519
(71) Anmelder: Zebris Medical GmbH, 88316 Isny im Allgäu (DE)
(72) Erfinder: Brunner, Wolfgang, 88316 Isny (DE)
(74) Vertreter: Meissner, Bolte & Partner

(57) **Zusammenfassung**

Abbildungsvorrichtung zur großflächigen Abbildung eines Körperabschnitts eines Wirbeltiers, insbesondere des Rückens oder der Extremitäten eines Menschen, mit Navigationsmitteln zur Positionsbestimmung abgebildeter Körperpunkte, aufweisend eine optische Aufnahmeeinrichtung zur Erzeugung von Bilddaten der Körperoberfläche, eine Bilddaten-Verarbeitungseinheit zur Erzeugung eines Primär-Skelettbildes der Knochen- und/oder Gelenkstruktur des Körperabschnitts aus den Bilddaten der Körperoberfläche und gespeicherten Standard-Skelettbildern, einen Abtastkopf zur schrittweise abtastenden Gewinnung eines Tiefen-Abtastbildes des Körperabschnitts und/oder einen Taster zum Antasten knöcherner Referenzen im Körperabschnitt, Markierungsmittel zur Markierung der Lage und Orientierung des Abtastkopfes und/oder des Tasters im Raum und eine Navigations-Basiseinrichtung zur räumlichen Positionserfassung der Markierungsmittel und eine Abtastdaten-Verarbeitungseinheit zur Gewinnung eines korrigierten Skelettbildes und/oder von anatomischen Drehpunkten des Körperabschnitts aufgrund des Primär-Skelettbildes und des mit Positionsdaten versehenen Tiefen-Abtastbildes und/oder der Positionsdaten der mit dem Taster angetasteten knöchernen Referenzen.

## Beschreibung

Die Erfindung betrifft eine Abbildungsvorrichtung zur großflächigen Abbildung eines Körperabschnitts eines Wirbeltiers, insbesondere des Rückens eines Menschen, auch bekannt als 3D-Rückenscanner,

Derartige Geräte sind seit längerem bekannt und werden unter anderem von der Anmelderin angeboten. Informationen zu Geräten dieser Art sind etwa unter www.diers.de zu finden. Meist werden hierbei Streifenlichtmuster auf den Rücken projiziert. Aus der 3D-Oberfläche können dann bestimmte Merkmale, z.B. die Lumbalgrübchen, bestimmt und diverse Parameter herausgerechnet werden. U.a. wird versucht, anhand der Hautoberfläche Rückschlüsse auf knöcherne Strukturen (z.B. Ort und Form der Wirbelsäule oder des Beckens) zu bestimmen. Sind Merkmale an der Hautoberfläche nicht erkennbar, so werden Markierungspunkte aufgeklebt und zur weiteren Bestimmung herangezogen. Ein Nachteil dieses Verfahrens ist es, dass die Hautoberfläche und die darunterliegenden Weichteile stark verschieblich und je nach Konstitution des Patienten sehr ungenau zu erfassen sind und damit eine sehr große Unsicherheit bei der Rekonstruktion knöcherner Strukturen besteht.

Bekannt und im klinischen Einsatz sind andererseits Systeme zur Positionsbestimmung von Körperabschnitten oder -teilen von Wirbeltieren, die auch als Navigationssysteme bezeichnet werden. Aus der umfangeichen Patentliteratur zu diesen Systemen wird im Hinblick auf die vorliegende Lösung speziell hingewiesen auf die DE 196 49 399 A1 und die DE 103 10 331 B3 der Anmelderin, die beide spezielle Vorrichtungen zur Messung der Beweglichkeit des Rückens bzw. von Funktionssegmenten der Wirbelsäure eines Menschen offenbaren. In der DE 196 49 399 A1 werden hierzu mit Gurten am Rücken des Menschen befestigte Halterungen mit Markierungsmitteln benutzt, die im Zusammenwirken mit einer stationären Messstation aufgrund von Laufzeitmessungen eine exakte und auch dynamische Positionsbestimmung des jeweiligen Körperabschnitts erlauben. Die DE 103 10 331 B3 lehrt den Einsatz eines mit ähnlichen Markierungsmitteln versehenen Ultraschall-Tastkopfes zur Untersuchung des Rückens eines Patienten, dessen exakte räumliche Position nach dem gleichen Prinzip bestimmt wird, Dieses System ermöglicht insbesondere die Erzeugung präziser Abbildungen der Oberflächenkonturen von Dorn- bzw. Querfortsätzen der Wirbelsäure und letztlich eine Bestimmung von deren Beweglichkeit.

Der Erfindung liegt die Aufgabe zugrunde, eine verbesserte Vorrichtung in Art des erwähnten 3D-Rückenscanners bereit zu stellen, welche insbesondere die Gewinnung genauerer Bilder von (speziell knöchernen) Strukturen unterhalb der Körperoberfläche und somit verbesserte diagnostische Aussagen ermöglicht.

Diese Aufgabe wird durch eine Abbildungsvorrichtung mit den Merkmalen des Anspruchs 1 gelöst. Zweckmäßige Fortbildungen des Erfindungsgedankens sind Gegenstand der abhängigen Ansprüche.

Die vorgeschlagene Vorrichtung umfasst zunächst - in an sich bekannter Weise - eine großflächig abbildende, insbesondere optische, Aufnahmeeinrichtung zur Erzeugung von Bilddaten der Körperoberfläche und eine Bilddaten-Verarbeitungseinheit zur Erzeugung eines Primär-Skelettbildes der Knochenstruktur des Körperabschnitts aus den Bilddaten der Körperoberfläche und gespeicherten Standard-Skelettbildern. Neben optischen Aufnahmeeinrichtungen, wie den auf diesem Gebiet bekannten 3D-Rückenscannern oder auch Laserscannern oder hochauflösenden Kameras, kommen zur Erzeugung der großflächigen Abbildung grundsätzlich etwa auch bildgebende Ultraschallverfahren oder Infrarotkameras oder auch mechanische Abtastvorrichtungen in Betracht, deren Datensätze zu einem Gesamtbild der interessierenden Körperoberfläche zusammengesetzt werden können. Standard-Skelettbilder bzw. entsprechend anatomische Datensätze sind im medizinischen Datenbasen verfügbar und können für die Nutzung in der vorgeschlagenen Vorrichtung intern gespeichert werden, oder es können auch Patienten spezifischer Datensätze (die etwa mittels anderer bildgebender Verfahren gewonnen wurden) gespeichert sein und verwendet werden.

Zusätzlich umfasst die Vorrichtung einen Abtastkopf zur schrittweise abtastenden Gewinnung eines Tiefen-Abtastbildes des Körperabschnitts und/oder einen Taster zum Antasten knöcherner Referenzen bzw. von Gelenkpunkten im Körperabschnitt und Markierungsmittel zur Markierung der Lage und Orientierung des Abtastkopfes und/oder des Tasters im Raum und eine Navigations-Basiseinrichtung zur räumlichen Positionserfassung der Markierungsmittel. Des Weiteren ist eine Abtastdaten-Verarbeitungseinheit zur Gewinnung eines korrigierten Skelettbildes bzw. von Koordinaten anatomischer Drehpunkte des Körperabschnitts aufgrund des Primär-Skelettbildes und des mit Positionsdaten versehenen Tiefen-Abtastbild und/oder der Positionsdaten der mit dem Taster angetasteten knöchernen Referenzen vorgesehen.

Dieses System, von dem einzelne Teile bei bestimmten Anwendungen auch fortgelassen sein und insbesondere die Bilddaten-Verarbeitungseinheit bzw. die Abtastdaten-Verarbeitungseinheit bzw. die Abtastdaten-Verarbeitungseinheit auch durch system-externe Verarbeitungskapazität, etwa auf einem PC oder PDA etc., ersetzt sein können, leistet demnach eine mehrstufige Gewinnung eines genauen Skelettbildes (oder allgemeiner auch Tiefenstrukturbildes) des Körperabschnitts. Hierzu werden Oberflächen-Bilddaten aus der großflächigen Abbildung mit Tiefen-Bilddaten und/oder Positionsdaten einzelner Skelett- bzw. Strukturpunkte positionsgetreu verknüpft.

In einer Ausführung der Erfindung ist der Abtastkopf als Ultraschall-Abtastkopf ausgebildet, und der Navigations-Basiseinrichtung ist eine Kalibrierungseinheit zur Kalibrierung der dem mit Tiefen-Abtastbild verknüpften Positionsdaten des Abtastkopfes anhand von dessen Abbildungscharakteristik zugeordnet. Bei dieser Ausführung wird also auf ein seit langem in der klinischen Praxis bewährtes und in hoher Qualität verfügbares Gerät zur Gewinnung der Tiefen-Bilddaten zurückgegriffen, und es werden diesem geeignete Mittel zur Positionszuordnung der gewonnenen Daten bzw. Bilder hinzugefügt.

In einer anderen Ausführung wird auf die Gewinnung von Tiefen-Bilddaten verzichtet, und statt dessen werden durch Antasten definierter Punkte der zu untersuchenden Struktur, speziell hervorgehobener knöcherner Referenzen, mit einem Taster unter gleichzeitiger Positionsbestimmung des Tasters den Bilddaten der Körperoberfläche bzw. dem daraus gewonnen Primär-Skelettbild nur ausgewählte Positionsdaten überlagert. Dies ermöglicht ebenfalls eine präzisierende Weiterverarbeitung des Struktur- bzw. Skelettbildes bzw. entsprechender Rohdaten zu anatomischen Drehpunkten, die bestimmten diagnostischen Anforderungen durchaus genügen kann.

Beide genannten Ausführungen sind auch kombinierbar und ergeben in der Kombination einen besonders hohen Grad an Bildgenauigkeit und diagnostischer Relevanz.

In einer weiteren Ausführung der Erfindung sind Markierungsmittel ("zweite Markierungsmittel") in den Abtastkopf und/oder Taster integriert oder mit diesem fest verbindbar. Hierbei können Markierungsmittel durch spezifisch geformte Oberflächenabschnitte des Abtastkopfes und/oder Tasters gebildet und die Navigations-Basiseinrichtung zur Erkennung der spezifisch geformten Oberflächenabschnitte und zur Erfassung ihrer Position ausgebildet sein. Es versteht sich, dass alternativ oder auch zusätzlich hierzu an sich bekannte Lokatoren ("Dreibeine") eingesetzt werden können.

In einer weiteren Ausführung, die insbesondere beim ausschließlichen Einsatz eines Tasters zur Verfeinerung des ursprünglich gewonnenen Bildes zweckmäßig ist, sind separate Referenz Markierungsstifte ("dritte Markierungsmittel") mit Fixierungsmitteln zur stationären Verbindung mit dem Körper vorgesehen. Die Positionen des Tasters können dann in Relation zu den Positionen der zusätzlichen Markierungsstifte gesetzt werden, um kleine Körperbewegungen in der verarbeitenden Berechnung zur Gewinnung des korrigierten Skelettbildes zu berücksichtigen. Speziell zum gleichzeitigen Antasten einander entsprechender Referenzen bzw. Gelenkpunkt an beiden Armen oder beiden Beinen kann ein speziell in Art einer Antastzange oder eines Abtastzirkels ausgebildeter Taster vorgesehen sein, der natürlich ebenfalls mit Markierungsmitteln versehen ist. Die Anbringung am Körper kann durch Aufkleben, durch Saugnäpfe oder auch über geeignete Bänder oder Gurte erfolgen, die den Körper im zu untersuchenden Bereich umschlingen und an denen die zusätzlichen Lokatoren geeignet fixiert werden können.

In weiteren Ausführungen der Erfindung weisen die Markierungsmittel auf Ultraschall- oder optischer oder magnetometrischer Basis arbeitende Sende- oder Empfangseinheiten oder Reflektoren und die Navigations-Basiseinrichtung eine hierzu korrespondierende Empfangs- und/oder Sendeeinheit auf. Derartige Markierungsmittel (Lokatoren) und mit ihnen zusammen wirkende Basisgeräte sind an sich bekannt und werden unter anderem von der Anmelderin angeboten, Dies gilt ebenso für eine zweckmäßige Ausführung der optischen Aufnahmeeinrichtung als Streifenlicht-Scanner. Die Aufnahmeeinrichtung kann daneben auch als Laserscanner ausgeführt sein, oder es kommt eine Kameraeinrichtung, insbesondere 3D-Kamera, zum Einsatz.

Während in bestimmten Ausführungen der Erfindung die Komponenten zur Positionsbestimmung als separates Teilsystem neben der großflächig abbildenden Aufnahmeeinrichtung vorgesehen sind, ist in einer speziellen Ausführung die Aufnahmeeinrichtung so angeordnet und dazu ausgebildet, zugleich als Navigations-Basiseinrichtung zur Positionserfassung der Markierungsmittel zu wirken. Dies vereinfacht insgesamt den gerätetechnischen Aufbau und die Handhabung. Im Sinne des oben erwähnten Systemaufbaus mit zusätzlichen Referenzen können hierbei insbesondere erste Markierungsmittel an einem handgeführten Taststift und zweite, als Referenz-Markierungsmittel dienende Markierungsmittel zur temporären festen Verbindung mit dem Körper vorgesehen sein. Bei Einsatz einer optischen Aufnahmeeinrichtung kommen entsprechend optische Markierungen zum Einsatz, während eine großflächig wirkende Ultraschall-Aufnahmeeinrichtung natürlich den Einsatz von Ultraschall-Sendeelementen oder -reflektoren erfordert.

In einer Ausgestaltung der vorgeschlagenen Vorrichtung speziell zur Analyse des menschlichen Ganges ist vorgesehen, dass die großflächig abbildende Aufnahmeeinrichtung jeweils mindestens eine auf beiden Seiten des Wirbeltieres zur Abbildung von dessen Armen und/oder Beinen oder von Abschnitten hiervon aufgestellte 3D-Kamera aufweist, wobei die rechts- und linksseitigen Kameras Mittel zur Herstellung einer definierten Lagebeziehung zwischen ihnen aufweisen. Noch spezieller ist dies dahingehend ausgestaltet, dass rechts- und linksseitig einer vorbestimmten Bewegungsrichtung des Wirbeltiers jeweils eine Mehrzahl von 3D-Kameras in vorbestimmten Abständen platziert ist. Eine noch weitere Ausführung sieht vor, dass zusätzlich zu den rechts- und linksseitigen 3D-Kameras rückseitig eine 3D-Kamera zur Abbildung der Rückseite des Wirbeltiers oder eines Abschnitts hiervon aufgestellt ist, welche bevorzugt ebenfalls Mittel zur Herstellung einer definierten Lagebeziehung zu den rechts- und linksseitigen Kameras aufweist.

Bei Einsatz eines Abtastkopfes zur Gewinnung eines Tiefen-Bildes des untersuchten Körperabschnitts, speziell eines Ultraschall-Abtastkopfes, muss eine möglichst exakte Beziehung zwischen der mit der Positionsbestimmungs-Komponente des Systems bestimmbaren Position des Abtastkopfes und der räumlichen Lage des durch ihn erzeugten Tiefen-Bildes hergestellt werden. Hierzu dient die weiter oben erwähnte Kalibrierungseinheit, und die von dieser benötigten Daten werden in einer Ausführung der Erfindung durch ein spezielles Kalibrierungsgerät als Vorrichtungskomponente gewonnen.

Vorteile und Zweckmäßigkeiten der Erfindung ergeben sich im Übrigen aus der nachfolgenden Beschreibung von Ausführungsbeispielen und -aspekten anhand der Figuren. Von diesen zeigen:
Fig. 1 eine Gesamtdarstellung einer Ausführungsform der erfindungsgemäßen Vorrichtung in Art eines Blockschaltbildes,
Fig. 2A und 2B Darstellungen zur Verdeutlichung der Funktion der Vorrichtung nach Fig. 1,
Fig. 3 eine perspektivische Ansicht eines Streifenlichtprojektors mit integrierter Navigations-Basiseinrichtung, wie er in einer ähnlichen Vorrichtung wie in der Fig. 1 gezeigten eingesetzt werden kann,
Fig. 4 eine perspektivische Darstellung eines Ultraschall-Abtastkopfes mit Markierungsmitteln zum Einsatz in der Vorrichtung nach Fig. 1 und einer ähnlichen Vorrichtung,
Fig. 5 eine schematische perspektivische Darstellung einer weiteren Ausführungsform der erfindungsgemäßen Vorrichtung und
die Figuren 6A und 6B skizzenartige Darstellung weiterer Ausgestaltungen der Erfindung.

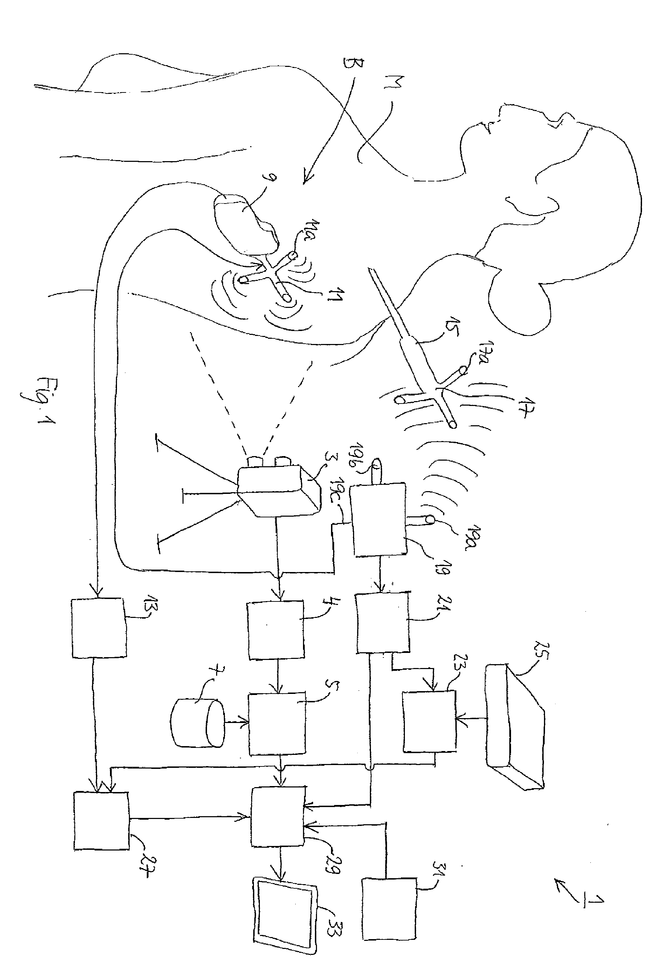

Fig. 1 zeigt schematisch den Aufbau einer Abbildungsvorrichtung 1 zur großflächigen Abbildung eines Körperabschnitts B eines Menschen M gemäß einer Ausführungsform der Erfindung. Ein stationär gegenüber dem Menschen M angeordneter Streifenlichtscanner 3 erzeugt in abtastender Weise ein Gesamtbild der Oberfläche des Rückens B bzw. eine Gesamtheit entsprechender Bilddaten und übergibt diese einer Vorverarbeitungseinheit 4, wo ein Oberflächenbild des Rückens B synthetisiert wird. Dieses Bild gelangt zu einer Bilddaten-Verarbeitungseinheit 5 zur Erzeugung eines Primär-Skelettbildes der Knochenstruktur des Rückens B unter Zugriff auf in einer Datenbasis 7 gespeicherte Standard-Skelettbilder bzw. entsprechende anatomische Daten.

Des Weiteren umfasst die dargestellte Vorrichtung einen Ultraschall-Abtastkopf 9 mit angebautem Lokator 11 mit drei Ultraschall-Sendeeinheiten 11a, dessen Datenausgang mit einer Ultraschallbild-Erzeugungseinheit 13 verbunden ist. Des Weiteren gehört zur Vorrichtung ein Taster 15 mit integriertem Lokator 17, welcher (abweichend vom Lokator 11 vom Ultraschall-Abtastkopf 9) als Markierungselemente Ultraschallreflektoren 17a trägt. Grundsätzlich erfordert der Einsatz des Tasters 15 mit integriertem Lokator zur verfeinerten Positionsbestimmung bestimmter anatomischer hervorgehobener Punkte (wie oben erläutert) das zusätzliche Anbringen von Referenz-Markierungsmüteln am Körper des Patienten; aus Gründen der besseren Übersichtlichkeit sind solche Referenz-Markierungsmittel in der Figur aber nicht dargestellt. Zu deren Ausführung und Anbringung kann etwa auf die DE 196 49 399 A1 verwiesen werden.

Mit den Lokatoren 11 und 17 arbeitet eine Navigations-Basiseinrichtung 19 zusammen, die einerseits über einen Ultraschallsender 19a zum Aussenden von Ultraschallsignalen an den Lokator 17 und zum anderen über einen Ultraschallempfänger 19b zum Empfang von Ultraschallsignalen von den Sendelementen 11a des Lokators 11 bzw. reflektierten Ultraschallsignalen von den Reflektorelementen 17a des Lokators 17 aufweist. Des Weiteren hat die Navigations-Basiseinrichtung 19 eine Leitungs-Verbindung 19c zum Lokator 11, um dessen Sendeelemente 11a zu versorgen und in geeigneter Synchronisation zu steuern. Die räumliche Position der Navigations-Basiseinrichtung 19 bezüglich des Streifenlichtscanners 3 muss durch den Vorrichtungsaufbau festgelegt sein, um die Positionsdaten korrekt mit den Bilddaten der Rückenoberfläche korrelieren zu können. Die geeignet synchronisierte Erzeugung und geordnete Verarbeitung der Sende- bzw. Ansteuersignale einerseits und der Empfangssignale andererseits in der Navigations-Basiseinrichtung 19 erfolgt in grundsätzlich bekannter Weise, so dass eine entsprechende Beschreibung hier nicht erforderlich ist.

Die in der Navigations-Basiseinrichtung 19 vorverarbeiteten Empfangsdaten gelangen zu einer Positionsdaten-Bestimmungseinheit 21, die die räumliche Position des Ultraschall-Abtastkopfes 9 bzw. Tasters 15 bestimmt. Sofern es sich um Positionsdaten des Ultraschall-Abtastkopfes 9 handelt, werden die Daten einer Kalibrierungseinheit 23 vorgeführt, welche zudem Daten aus einem Kalibrierungsgerät 25 empfängt und kalibrierte Positionsdaten liefert, welche direkt dem durch den Ultraschall-Abtastkopfes 9 gewonnenen Tiefen-Abtastbild des Rückens B zugeordnet werden können. Diese Zuordnung erfolgt in einer Ultraschalldaten-Verknüpfungseinheit 27. Die den Taster 15 betreffenden Positionsdaten hingegen werden keiner zusätzlichen Verarbeitung unterzogen.

Das Kalibrierungsgerät 25 dient zur Lieferung von die Abbildungscharakteristik des Abtastkopfes 9 kenzeichnenden Daten, mit denen eine Umrechnung der Abtastkopf gewonnenen Positionsdaten in dem Ultraschallbild zuzuordnenden Positionsdaten erfolgt. Eine Ausführung des Kalibrierungsgerätes kann so aussehen, dass auf einer Halteplatte feste Markierungspunkte (Ultraschallsender) sowie ein Wasserbad angebracht sind, in das der Abtastkopf eingetaucht und dem er zum Zwecke der Kalibrierung in Betrieb genommen wird. Das erzeugte Ultraschallbild wird aufgenommen und der Abtastkopf über Einstellschrauben so lange verstellt, bis ein im Wasserbad in fester räumlicher Relation zu den Ultraschallsendern eingelassener Metallstift klar im Ultraschallbild (Schnittbild) erkennbar ist. Durch Bestimmung der Positionsdaten der hier als Referenz dienenden Ultraschallsender, der am Lokator des Abtastkopfes selbst angebrachten Markierungsmittel und der bekannten räumlichen Relation zwischen dem Metallstift und den Ultraschallsendern des Kalibrierungsgerätes kann eine hinreichend genaue Relation zwischen den Positionsdaten des Abtastkopfes und dem Ultraschallbild bzw. dem Tiefen-Bilddaten hergestellt werden.

Die Bilddaten des Primär-Skelettbildes aus der Bilddaten-Verarbeitungseinheit 5 werden mit den Positionsdaten des Tasters 15 und den kalibrierten Positionsdaten der Ultraschallbilder (Tiefenabtastbilder) in einer Abtastdatenverarbeitungseinheit 29 zusammengeführt. In diese gelangen auch Eingaben aus einer Eingabeeinheit 31, etwa zur Benennung einer mit dem Taster 15 aktuell angetasteten knöchernen Referenz im Körperabschnitt (Rücken) B. Zur Gewinnung des gewünschten verfeinerten (korrigierten)-Skelettbildes werden aus den Ultraschallbildern durch an sich bekannte Algorithmen und mittels bekannter Software die knöchernen Randkonturen erkannt, die Ultraschallbilder können durch den Arzt aber auch entsprechend manuell bearbeitet werden. Eine durchgehende Abtastung des Rückens mit dem Abtastkopf ist im Übrigen nicht erforderlich.

In der Abtastdaten-Verarbeitungseinheit 29 wird aufgrund all dieser Daten ein korrigiertes Skelettbild des Rückens errechnet, und dieses kann auf einer Anzeigeeinheit 33 angezeigt werden. (Es versteht sich, dass auch die im Ergebnis der verschiedenen Vorverarbeitungen gewonnenen Bilder angezeigt werden können, entsprechende Signalverbindungen sind aber im Interesse der besseren Übersichtlichkeit in der Figur nicht dargestellt.)

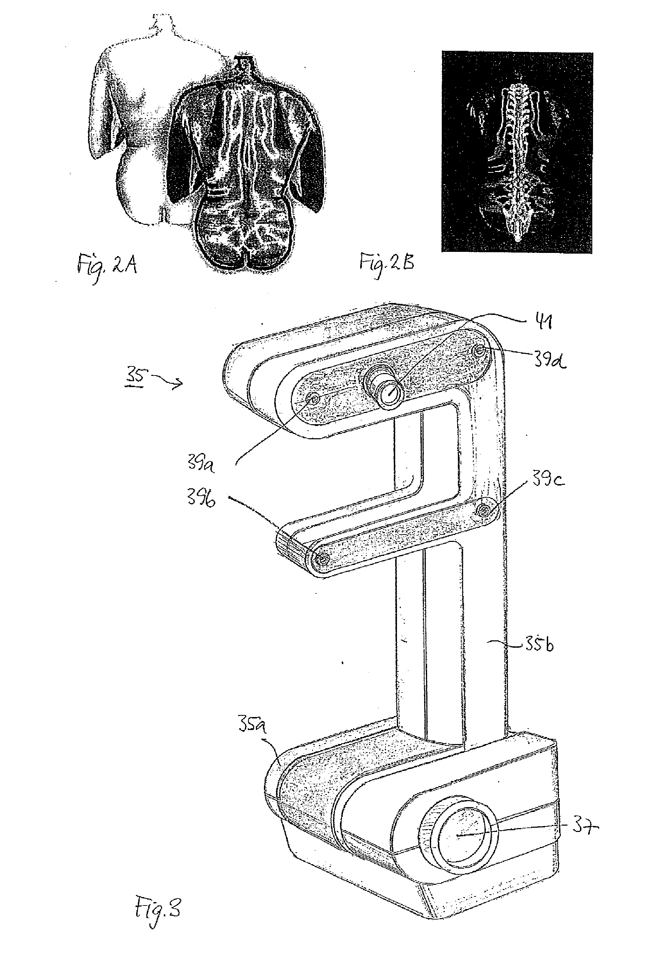

Fig. 2A und 2B zeigen zur Illustrierung der Funktionsweise der Vorrichtung 1 einerseits ein Bild der Körperoberfläche, zusammen mit einer "Krümmungs-Karte" des Rückens, die zur Visualisierung anatomischer Orientierungspunkte (Landmarks) dient, und andererseits ein Skelettbild bzw. Tiefenstrukturbild, wie es typischerweise am Ende des Verarbeitungsprozesses der Vorrichtung stehen kann.

Fig. 3 zeigt ein Kombinationsgerät 35, welches die Funktionskomponenten eines Streifenlichtscanners sowie einer Navigations-Basiseinrichtung in sich vereinigt und in einer ähnlichen Vorrichtung wie in der in Fig. 1 gezeigten grundsätzlich die Komponenten Streifenlichtscanner 3 und Navigations-Basiseinrichtung 19 ersetzen könnte. Auf einen Grundkörper 35a, der einen Streifenlichtprojektor 37 aufnimmt, ist ein ständerartiger Fortsatz 35b aufgesetzt, der in zwei übereinander liegenden Plattformen einerseits vier Ultraschallempfänger 39a bis 39d einer (im Übrigen nicht separat zu erkennenden) Navigations-Basiseinrichtung sowie zusätzlich eine Kamera 41 trägt. Da sowohl die Funktionsweise eines Streifenlichtscanners als auch diejenige einer Navigations-Basiseinrechtung der hier einsetzbaren Art sowie diejenige einer Kamera bekannt sind, wird von einer weitergehenden Beschreibung Abstand genommen.

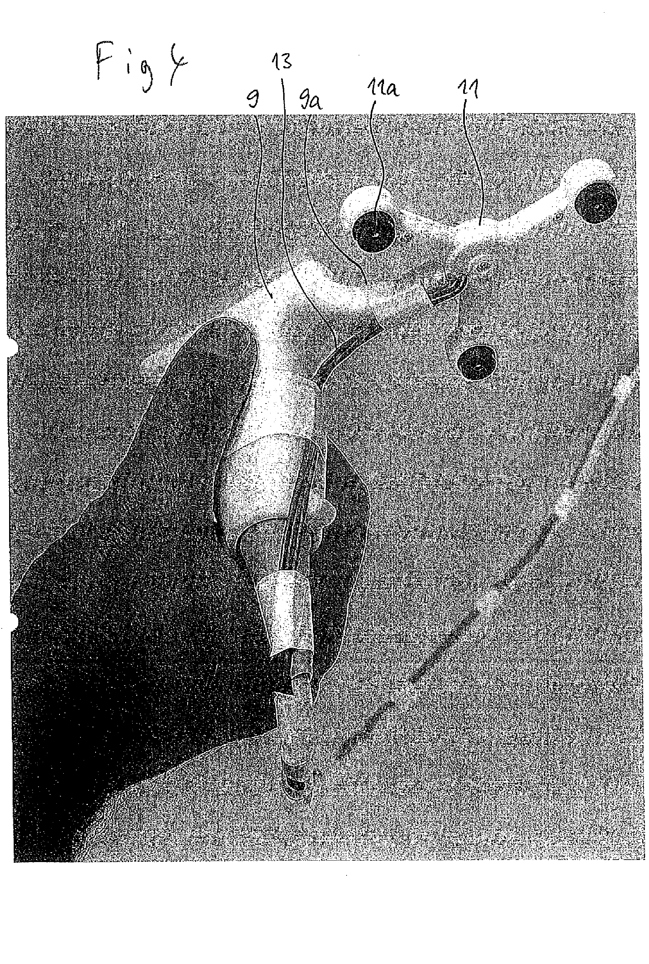

Fig. 4 zeigt in einer perspektivischen Darstellung eine Ausführungsform des Ultraschall-Abtastkopfes 9 mit angesetztem Lokator 11 genauer. Es ist zu erkennen, dass der Lokator 11 mit den Navigationsmitteln 11a (welche grundsätzlich Empfänger oder Sender oder auch Reflektoren sein und mit entsprechenden korrespondierenden Sende- bzw. Empfangselementen an einer Navigations-Basiseinrichtung zusammenwirken können) derart an einem Fortsatz 9a des Abtaster-Gehäuses angebracht ist, dass die Handhabung durch den Arzt die Ultraschallübertragung nicht behindert. Anschlussleitungen 13 des Lokators 11 sind soweit als möglich am Gehäuse des Abtasters 9 entlang geführt.

Die Ausführung der Erfindung ist nicht auf die hier gezeigten und beschriebenen Beispiele und der hervorgehobenen Aspekten beschränkt, sondern ebenso in einer Vielzahl von Abwandlungen möglich, die im Rahmen fachgemäßen Handelns liegen. Insbesondere sei nochmals daraufhin gewiesen, dass an Stelle eines herkömmlichen Streifenlichtscanners auch ein Laserscanner oder eine Kameraeinrichtung mit geeigneter Leistungsparametern vorgesehen sein kann und dass die optische Aufnahmeeinrichtung, geeignete Mittel zur Bildverarbeitung vorausgesetzt, zugleich als Navigations-Basiseinrichtung ausgebildet sein kann und in diesem Falle mit optisch wirkenden Markierungsmitteln (ggfs, auch markanten Gehäuseabschnitten des Abtastkopfes und/oder Tasters) zusammenwirkt und diese zur Gewinnung von Positionsdaten verarbeiten kann.

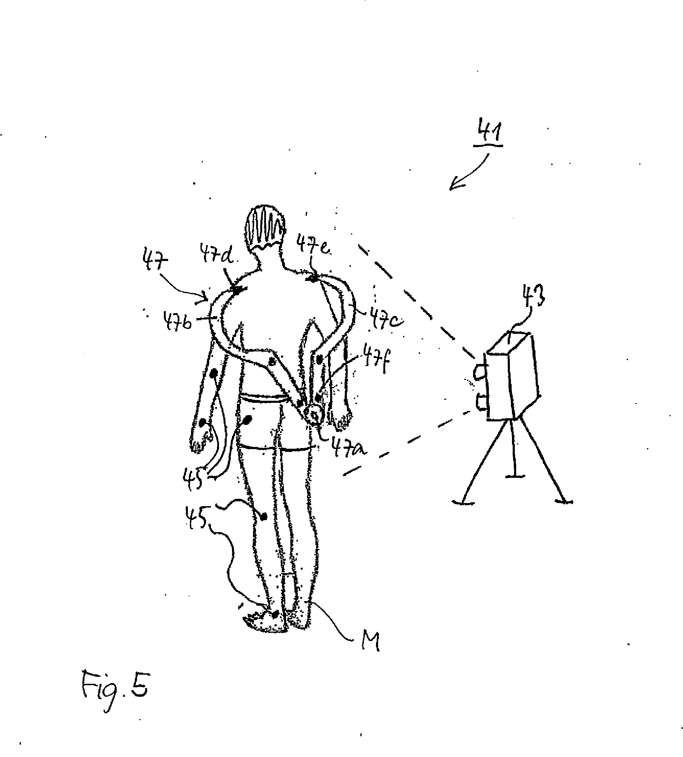

Fig. 5 zeigt skizzenhaft eine weitere erfindungsgemäße Abbildungsvorrichtung 41 im Einsatz an einem menschlichen Probanden M, die eine 3D-Kamera 43 als großflächig abbildende Aufnahmeeinrichtung umfasst und speziell zur Bestimmung anatomischer Drehpunkte 45 eines menschlichen Körpers und zu dessen anatomisch weitgehend korrekter Darstellung in Bewegung ausgebildet ist. Bei den anatomischen Drehpunkten handelt es sich, wie aus der Figur zu entnehmen, insbesondere um das Hüftgelenk sowie Knie-, Sprung-/Fuß- sowie Schulter- und Armgelenke des Probanden M. Diese Drehpunkte 45 können aus einem reinen 3D-Oberflächenbild bei stehendem Probanden - speziell bei Probanden mit Bewegungseinschränkungen - nicht hinreichend erfasst werden, oder die Bestimmung ist mit sehr hohem Aufwand verbunden. Zusätzliche Schwierigkeiten ergeben sich, wenn Kleidungsstücke für eine zusätzliche unscharfe der Körperkontur sorgen.

Möglich und sinnvoll ist jedoch eine mechanische Antastung, verbunden mit einer "Navigation". Hierzu kann ein weiter oben erwähnter Taststift genutzt werden; in der Figur ist jedoch eine Abtastzange 47 als speziell konstruierter Taster dargestellt, mit der jeweils einander entsprechende rechts- und linksseitige Drehpunkte 45 gleichzeitig angetastet werden können. Die Abtastzange 47 umfasst zwei betragsgleich um eine Drehachse 47a schwenkbare Arme 47b, 47c, an deren freien Enden sich jeweils eine Tastspitze 47d, 47e befindet. Auf die Arme 47b, 47c aufgesetzte Navigations-Markierungspunkte 47f ermöglichen die Bestimmung der jeweiligen räumlichen Lage der Abtastzange, wahlweise durch ein separates (in der Figur nicht dargestelltes) Positionsbestimmungssystem an sich bekannter Art oder in einfacher Weise direkt mittels der 3D-Kamera 43. Aus den hiermit bestimmten Raumkoordinaten der Markierungspunkte 47f lassen sich durch einfache Offset-Berechnung die Raumkoordinaten der Tastspitzen 47d, 47e und somit die Lage der jeweils angetasteten Drehpunkte des Körpers bestimmen.

Anhand dieser Raumkoordinaten kann in einer (hier ebenfalls nicht dargestellten) Verarbeitungseinheit ein virtuelles gelenkbezogenes Skelettmodel des Probanden M erzeugt werden, und in weiteren Verarbeitungsvorgängen können die gemessenen 3D-Oberflächendaten fortlaufend mit einem vorab bestimmten 3D-Skelettmodell bzw. den gemessenen Koordinaten der Drehpunkte 45 bestmöglich zur Deckung gebracht werden. Damit kann schließlich ein 3D-Modell des Probanden (z.B. als Skelettmodell) in Bewegung dargestellt werden, d.h. die Bewegungen des Probanden können anschließend ausschließlich aufgrund der fortlaufend mit der 3D-Kamera 43 gewonnenen Oberflächendaten bildlich dargestellt werden.

In einer verfahrensmäßigen Ausgestaltung kann im Übrigen auch während der Eingabe/Erfassung der anatomischen Drehpunkte mit der Abtastzange 47 (oder auch - etwas zeitaufwändiger - mit einem einfachen Taststift) ein Abtasten des Ausmessen der Körperoberfläche erfolgen. Hierbei können Veränderungen der Lage der Drehpunkte während des (einige Zeit erfordernden) Antast- bzw. Erfassungsvorganges berücksichtigt und ggfs. korrigiert werden. Derartige Lageveränderungen können bspw. dadurch zustande kommen, dass der Proband in der benötigten Zeitspanne leicht schwankt oder unwillkürliche Bewegungen ausführt.

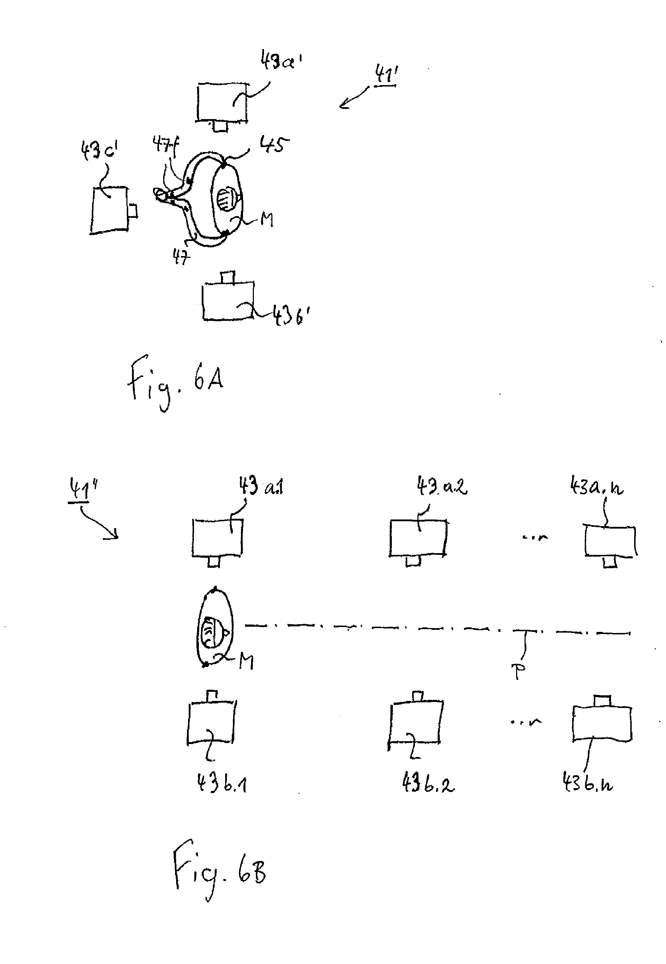

In den Figuren 6A und 6B sind skizzenartig Modifikationen der in Fig. 5 gezeigten Vorrichtung dargestellt. Fig. 6A zeigt hierbei eine Abbildungsvorrichtung 41', bei dem als großflächig abbildende Aufnahmeeinrichtung eine Gruppe von drei 3D-Kameras 43a', 43b', 43c' vorgesehen ist. Hiervon sind die Kameras 43a', 43b' rechts- bzw. linksseitig vom Probanden M positioniert, während die dritte Kamera 43c' in dessen Rücken steht. Wie bei der Ausführung nach Fig. 5 werden anatomische Drehpunkte 45 mittels der Tastzange 47 angetastet, und deren Marker 47f werden entweder von der linken oder rechten oder beiden seitlichen Kameras erfasst.

Die rückseitige Kamera 43c' (deren Vorsehen optional ist) kann bei einer anfänglichen Bestimmung der Raumkoordinaten der anatomischen Drehpunkte die Marker 47f auch allein erfassen, und die seitlichen Kameras übernehmen dann die Aufzeichnung von Bewegungen des Probanden. Es könnte sich dann bei diesen seitlichen Kameras um reine 3D-Abbildungseinrichtungen handeln, während die rückseitige Kamera speziell als Kamera eines Positionsbestimmungssystems ausgebildet sein und funktionieren könnte. Sofern eine weitere (nicht dargestellte) Aufnahmeeinrichtung eines Positionsbestimmungssystems zur Erfassung der Marker-Signale vorgesehen ist, können selbstverständlich auch alle drei Kameras reine Oberflächen-Abbildungseinrichtungen sein.

Mit der zuletzt beschriebenen Anordnung können unter Umständen Bein- und Armbewegungen besser erfasst werden. Wesentlich ist, dass insbesondere die seitlich angeordneten Kameras 43a', 43b' in definierter Relativposition zueinander angeordnet sind, so dass durch beide Kameras ein definierter Messraum aufgespannt wird. Im einfachsten Fall wird hierzu nur der Abstand zwischen den beiden Kameras ermittelt, eine bevorzugte Ausführung sieht jedoch eine kalibrierte Definition des erwähnten Messraumes durch Auswertung der Signale von Markern vor, die im Blickfeld beider Kameras liegen.

Die weiter modifizierte Abbildungsvorrichtung 41", die in Fig. 6B skizziert ist, umfasst jeweils eine ganze Gruppe von linksseitigen Kameras 34a.1 bis 34a.n und rechtsseitigen Kameras 34b.1 bis 34b.n, die wiederum in vorbestimmter Lagebeziehung zueinander längs eines Fortbewegungs-Weges P des Probanden M positioniert sind. Diese Ausgestaltung eignet sich zur Verfolgung des Ganges des Probanden über eine längere Wegstrecke. Es versteht sich, dass auch bei dieser Ausgestaltung eine Kalibrierung zur Bestimmung eines definierten Messraumes erforderlich ist. Derartige Bestimmungen/Kalibrierungen sind dem Fachmann für Positionsbestimmungssysteme an sich bekannt und werden daher hier nicht weiter erläutert.

## Patentansprüche

1. Abbildungsvorrichtung zur großflächigen Abbildung eines Körperabschnitts eines Wirbeltiers, insbesondere des Rückens oder der Extremitäten eines Menschen, mit Navigationsmitteln zur Positionsbestimmung abgebildeter Körperpunkte, aufweisend
eine optische Aufnahmeeinrichtung zur Erzeugung von Bilddaten der Körperoberfläche,
eine Bilddaten-Verarbeitungseinheit zur Erzeugung eines Primär-Skelettbildes der Knochen- und/oder Gelenkstruktur des Körperabschnitts aus den Bilddaten der Körperoberfläche und gespeicherten Standard-Skelettbildern,
einen Abtastkopf zur schrittweise abtastenden Gewinnung eines Tiefen-Abtastbildes des Körperabschnitts und/oder einen Taster zum Antasten knöcherner Referenzen im Körperabschnitt,
Markierungsmittel zur Markierung der Lage und Orientierung des Abtastkopfes und/oder des Tasters im Raum und eine Navigations-Basiseinrichtung zur räumlichen Positionserfassung der Markierungsmittel und
eine Abtastdaten-Verarbeitungseinheit zur Gewinnung eines korrigierten Skelettbildes und/oder von anatomischen Drehpunkten des Körperabschnitts aufgrund des Primär-Skelettbildes und des mit Positionsdaten versehenen Tiefen-Abtastbildes und/oder der Positionsdaten der mit dem Taster angetasteten knöchernen Referenzen.

2. Abbildungsvorrichtung nach Anspruch 1, wobei der Abtastkopf als Ultraschall-Abtastkopf ausgebildet und der Navigations-Basiseinrichtung eine Kalibrierungseinheit zur Kalibrierung der dem Tiefen-Abtastbild zugeordneten Positionsdaten des Abtastkopfes anhand der Abbildungscharakteristik des Abtastkopfes zugeordnet ist.

3. Abbildungsvorrichtung nach Anspruch 1 oder 2, wobei erste Markierungsmittel in den Abtastkopf und/oder Taster integriert oder mit diesem fest verbindbar sind.

4. Abbildungsvorrichtung nach Anspruch 3, wobei erste Markierungsmittel durch spezifisch geformte Oberflächenabschnitte des Abtastkopfes und/oder Tasters gebildet sind und die Navigations-Basiseinrichtung zur Erkennung der spezifisch geformten Oberflächenabschnitte und zur Erfassung ihrer Position ausgebildet ist.

5. Abbildungsvorrichtung nach einem der vorangehenden Ansprüche, wobei separate Referenz-Markierungsstifte mit Fixierungsmitteln zur stationären Verbindung mit dem Körper vorgesehen sind.

6. Abbildungsvorrichtung nach einem der vorangehenden Ansprüche, wobei die Markierungsmittel auf Ultraschall- oder optischer oder magnetometrischer Basis arbeitende Sende- oder Empfangseinheiten oder Reflektoren und die Navigations-Basiseinrichtung eine hierzu korrespondierende Empfangs- und/oder Sendeeinheit aufweisen.

7. Abbildungsvorrichtung nach einem der vorangehenden Ansprüche, wobei die großflächig abbildende Aufnahmeeinrichtung einen Streifenlicht- oder Laserscanner oder eine Kameraeinrichtung, insbesondere 3D-Kamera, aufweist.

8. Abbildungsvorrichtung nach einem der vorangehenden Ansprüche, wobei die großflächig abbildende Aufnahmeeinrichtung oder eine Komponente hiervon entfernt vom Körper angeordnet und dazu ausgebildet ist, zugleich als Navigations-Basiseinrichtung zur optischen Positionserfassung der Markierungsmittel zu wirken.

9. Abbildungsvorrichtung nach Anspruch 7 oder 8, wobei die großflächig abbildende Aufnahmeeinrichtung jeweils mindestens eine auf beiden Seiten des Wirbeltieres zur Abbildung von dessen Armen und/oder Beinen oder von Abschnitten hiervon aufgestellte 3D-Kamera aufweist, wobei die rechts- und linksseitigen Kameras Mittel zur Herstellung einer definierten Lagebeziehung zwischen ihnen aufweisen.

10. Abbildungsvorrichtung nach Anspruch 9, wobei rechts- und linksseitig einer vorbestimmten Bewegungsrichtung des Wirbeltiers jeweils eine Mehrzahl von 3D-Kameras in vorbestimmten Abständen platziert ist.

11. Abbildungsvorrichtung nach Anspruch 9 oder 10, wobei zusätzlich zu den rechts- und linksseitigen 3D-Kameras rückseitig eine 3D-Kamera zur Abbildung der Rückseite des Wirbeltiers oder eines Abschnitts hiervon aufgestellt ist.

12. Abbildungsvorrichtung nach Anspruch 8, wobei zweite optische Markierungsmittel an einem handgeführten Taststift oder einer handgeführten Abtastzange und dritte, als Referenz-Markierungsmittel dienende optische Markierungsmittel zur temporären festen Verbindung mit dem Körper vorgesehen sind.

13. Abbildungsvorrichtung nach einem der Ansprüche 2 bis 12, zusätzlich aufweisend
ein Kalibrierungsgerät zur Gewinnung der in der Kalibrierungseinheit zu verwendenden Abbildungscharakteristik des Ultraschall-Abtastkopfes.
